# EUROPÄISCHE PATENTANMELDUNG

(11) **EP 0 538 659 A2**
(43) Veröffentlichungstag der Anmeldung: **28.04.1993**
(21) Anmeldenummer: 92116850.6
(22) Anmeldetag: 01.10.1992
(51) Int. Cl.: A61B 17/22, A61B 6/12

(54) **Extrakorporales Therapiegerät**

(30) Priorität: 25.10.1991 DE 4135328
(71) Anmelder: Richard Wolf GmbH, D-75438 Knittlingen (DE)
(72) Erfinder: Bauer, Edgar, W-7527 Kraichtal 3 (DE); Krauss, Werner, W-7134 Knittlingen (DE); Meyer, Michael, W-7520 Bruchsal 3 (DE); Fladl, Joachim, W-7519 Oberderdingen 3 (DE)
(74) Vertreter: Vollmann, Heiko, Dipl.-Ing.

(57) **Zusammenfassung**

Das beschriebene Therapieger{t (1) zum Behandeln von Konkrementen und Gewebe im Kωrper von Patienten mittels Schallwellen umfaßt einen Schallwellengenerator (2) sowie eine beistellbare Rωntgeneinrichtung (9) zum Orten eines Therapieobjektes, wobei das Therapieger{t eine Zieleinrichtung (8) aufweist. Um eine bekannte und zum Therapieger{t beistellbare Rωntgeneinrichtung relativ zu dem Therapieger{t schnell und sicher genau justieren und fixieren zu kωnnen, ist die Zieleinrichtung (8) innerhalb des axialen Durchgangs eines am Schallwellengenerator (2) befestigten und mit seiner zentralen L{ngsachse (4a) auf dessen Fokus ausgerichteten Rωntgentubus (4) f}r die beistellbare Rωntgeneinrichtung (9) angeordnet.

## Beschreibung

Die Erfindung geht aus von einem extrakorporalen Therapiegerät zum Behandeln von Konkrementen und Gewebe mittels Schallwellen im Körper eines Patienten mit einer Generatoreinrichtung zum Erzeugen der Schallwellen, mit einer Zieleinrichtung und unter Verwendung einer beistellbaren Röntgeneinrichtung zum Orten eines Therapieobjektes.

In der EP-OS 0 402 584 ist ein Therapiegerät der vorstehenden Art in Form eines Lithotriptors beschrieben. Hierbei ist außen und direkt am Kopf eines Stoßwellengenerators eine Zieleinrichtung montiert, die aus einem Träger aus röntgennegativem Material und aus mehreren, darin eingelagerten Zielmarken aus röntgenpositivem Material besteht. Es sind vier Zielmarken bzw. Referenzpunkte vorgesehen, und jeweils zwei Zielmarken des genannten Trägers definieren je eine Zielgerade, so daß zwei gedachte Zielgeraden definiert sind. Dieser so ausgestattete Stoßwellengenerator wird in Verbindung mit einer beistellbaren Röntgeneinrichtung der C-Arm-Bauart verwendet. Die beiden Zielgeraden schneiden sich in einem Punkt, der mit dem Fokuspunkt des Stoßwellengenerators in Übereinstimmung gebracht werden muß und in den das spätere Therapieobjekt des Patienten eingestellt wird.

Obwohl diese Zieleinrichtung die Einstellung der Röntgeneinrichtung auf den Fokus des Stoßwellengenerators und damit auf ein Konkrement im Körper eines Patienten erleichtert, ist die Durchführung der Einstellung und damit der Ortung mit dieser Zieleinrichtung trotzdem noch umständlich und nicht sicher. Nach der groben Einstellung der beigestellten Röntgeneinrichtung auf die Zieleinrichtung muß zunächst das Strahlenbüschel der Röntgeneinrichtung mit der ersten Zielgeraden der Zieleinrichtung in Übereinstimmung gebracht werden. Sodann müssen eine fiktive Fokusstelle des Stoßwellengenerators auf einer ersten Geraden, die der ersten Zielgeraden entspricht, auf dem Bildschirm eines Monitors festgelegt und dann das ebenfalls auf dem Bildschirm sichtbare Therapieobjekt des Patienten auf den fiktiven Fokus eingestellt werden. Zum Erhalt dieses Ergebnisses werden mehrere Röntgenaufnahmen gemacht. Durch Verschwenkung der Röntgeneinrichtung in die zweite Zielgerade der Zieleinrichtung ist die dritte Raumkoordinate des Therapiepunktes des Patienten in bezug auf den fiktiven Fokus ersichtlich. Durch die übliche Verstellung des Liegetisches, auf dem sich der Patient befindet, wird dessen Therapieobjekt nun in die endgültige fiktive Fokusstelle eingestellt, wodurch der wahre Fokus des Stoßwellengenerators nun tatsächlich mit dem Therapieobjekt übereinstimmt. Auch für dieses Ergebnis werden mehrere Röntgenaufnahmen gemacht. Insgesamt ist hierbei die Röntgenstrahlenbelastung relativ hoch. Des weiteren ist die Zieleinrichtung ungeschützt und kann selbst leicht verstellt werden, z.B. durch Anstoßen.

In der DE-OS 40 07 669 ist ein Lithotriptor beschrieben, dessen Stoßwellengenerator mit einer Röntgeneinrichtung versehen ist, deren Strahlenachse mit dem Fokus des Generators zusammenfällt und die aus einem Röntgentubus und der eigentlichen Röntgenstrahlenquelle besteht. In der DE-OS 39 16 093 ist ein weiterer Lithotriptor beschrieben, der ebenfalls mit einer Röntgeneinrichtung der C-Arm-Trägerbauart, die in drei Raumkoordinaten einstellbar ist, fest verbunden ist. Diese Lithotriptoren haben einen speziellen C-Arm für die Röntgeneinrichtung , der mit dem Stoßwellengenerator ständig fest verbunden ist.

Die Aufgabe der Erfindung besteht darin, ein extrakorporalesa Therapiegerät der einleitend angeführten Art so zu verbessern, daß die Positionierung einer beistellbaren Röntgeneinrichtung zu dem Therapiegerät zwecks schnellerer Röntgenortung des Therapieobjektes des Patienten schnell, sicher und einfach möglich ist.

Die Lösung der Aufgabe ist in dem Patenanspruch 1 angegeben.

Durch diese Lösung ist die Ausrichtung der beistellbaren, in den Kliniken neben anderen Aufgaben z.B. auch für die Lithotripsie- und Gewebebehandlung verwendeten Röntgeneinrichtungen der C-Arm-Bauart auf den Fokus des Stoßwellengenerators und damit auf das mittels Ultraschall zu behandelnde Konkrement oder Gewebe des Patienten sowie die Röntgenortung des Therapieobjektes wesentlich erleichtert. Dies folgt aus der Tatsache, daß die zentrale Längsachse des Röntgentubus infolge der Festmontage dieses Tubus am Ultraschallgenerator von vornherein durch den Fokus des Generators verläuft, und die einzige Zielgerade der jetzt innenliegenden Zieleinrichtung des Röntgentubus mit dessen zentraler Längsachse zusammenfällt und daraus, daß die Röntgeneinrichtung konfokal mit dem Stoßwellengenerator verschwenkt wird, weil der die Röntgeneinrichtung tragende C-Arm-Träger an dem Stoßwellengenerator lösbar fixiert ist und C-Arm-Träger und Stoßwellengenerator als Einheit gemeinsam verschwenkt werden. Es entfällt somit eine gesonderte Einstellung des Röntgengerätes der C-Arm-Bauart zu einer zweiten Zielgeraden. Die Zielmarken der Zieleinrichtung erscheinen auf dem Beobachtungsmonitor bei richtiger Einstellung der Röntgeneinrichtung immer in Überdeckung, und das ebenfalls auf dem Monitor sichtbare Therapieobjekt kann leicht mittels des in den drei Raumkoordinaten X, Y und Z einstellbaren Patientenliegetisches in die abgebildeten Zielmarken eingestellt werden. Durch die erfindungsgemäße Anordnung der Zieleinrichtung geschützt innerhalb des Röntgentubus ist der Röntgenstrahl der beistellbaren Röntgeneinrichtung mit seiner Hauptchse auf einfache Weise auf den Fokus des Stoßwellengenerators ausgerichtet, so daß die Zieleinrichtung nach Justage und Fixierung der Röntgeneinrichtung am Stoßwellengenerator in jeder Projektionsrichtung exakt den geometrischen Ort des Fokus des Stoßwellengenerators auf dem Röntgenmonitor darstellt.

Eine bevorzugte Ausführungsform der Zieleinrichtung besteht aus zwei im Innern des Röntgentubus voneinander mit Abstand und parallel zueinander angeordneten Scheiben aus röntgennegativem Material und aus an oder in den Scheiben vorgesehenen Zielmarken aus röntgenpositivem Material, wobei die Mittelpunkte der Scheiben auf der Mittelachse des Röntgentubus liegen. Die Zielmarken selbst können z.B. aus in den Scheiben ausgebildeten, geometrische Figuren darstellenden Kanälen und aus einer durch die Kanäle pumpbaren, röntgenpositiven Flüssigkeit bestehen. Diese Flüssigkeit kann ein Gemisch aus Alkohol und Röntgenkontrastmittel sein. Diese Zieleinrichtung weist eine einfache Konstruktion auf und kann dadurch ausgeblendet werden, daß die besagte Flüssigkeit wieder aus den Kanälen herausgepumpt wird.

Die Erfindung ist nachstehend in Verbindung mit den anliegenden Zeichnungen beispielsweise erläutert. Es zeigen in schematischer Darstellung:
- Figur 1: einen vereinfachten Axialschnitt durch einen Stoßwellengenerator mit beigestelltem, teilweise gezeigtem C-Arm-Träger, wobei der Generator und der C-Arm-Träger zusammen als Einheit zueinander justiert sind,
- Figur 2: einen axialen, vergrößerten Teilschnitt des Stoßwellengenerators nach Fig. 1 mit einer ersten Ausführungsform einer Zieleinrichtung,
- Figur 3: ein System zum Betrieb der Zieleinrichtung nach Figur 2,
- Figur 4: einen axialen vergrößerten Teilschnitt einer zweiten Ausführungsform der Zieleinrichtung,
- Figur 5: eine Aufsicht auf die Darstellung nach Figur 4,
- Figur 6: eine Endansicht auf eine Gesamtanordnung aus Lithotriptor, Patientenliegetisch und beigestellter Röntgeneinrichtung,
- Figur 7: eine Seitenansicht auf die Gesamteinrichtung gemäß dem Pfeil A in Figur 6,
- Figur 8: eine Darstellung einer anderen Ausführungsform des Stoßwellengenerators mit beigestelltem C-Arm-Träger, die eine feste Einheit bilden,
- Figur 9: einen weiteren, vereinfachten Axialschnitt durch einen Stoßwellengenerator mit Ultraschallortungseinrichtung im Röntgentubus.

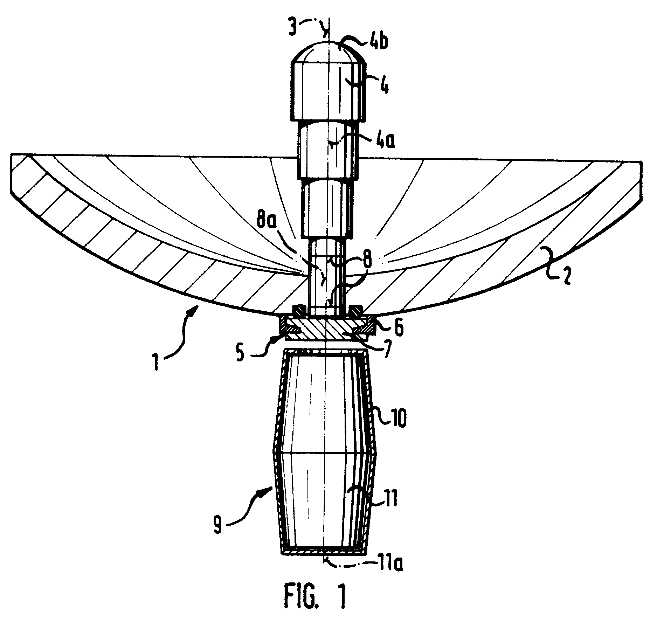

Figur 1 zeigt ein allgemein mit 1 bezeichnetes extrakorporales Therapiegerät zum Erzeugen von Schallwellen zwecks Behandlung eines Therapieobjektes eines Patienten, z.B. von Konkrementen oder Geweben. Die Behandlung mit Schallwellen erfolgt für Konkremente in der Regel mittels Ultraschall-Stoßwellen und für Gewebe, z.B. Tumore, mittels transienter hochenergetischer Ultraschallimpulse zur mechanischen Gewebezerreißung oder mittels hochenergetischem Ultraschall im Dauer- oder Burstbetrieb zur thermischen Gewebezerstörung.

Das Therapiegerät 1 umfaßt einen bekannten Schallwellengenerator 2 der Kalottenbauart, z.B. für Ultraschall-Stoßwellen. In dem durch die strichpunktierte Linie 3 angegebenen Zentrum des Generators 2 ist ein Röntgentubus 4 fest mit dem Generator verbunden. Dieser Röntgentubus ist vorzugsweise längenverstellbar ausgebildet, wie es zum Beispiel in der DE-A-40 07 669 angegeben ist, so daß sich eine nähere Erläuterung seiner Konstruktion erübrigt. Der untere Bereich des Röntgentubus 4 ist durch eine Verschlußeinrichtung 5 luftdicht abgeschlossen, die aus einem am Generator 2 befestigten Bajonettfassungsteil 6 und einem daran abnehmbar angeordneten Verschlußplattenteil 7 besteht. Das Plattenteil 7 ist für Röntgenstrahlen durchlässig. Die Längsachse 4a des Röntgentubus 4 fällt mit der zentralen Achse 3 des Generators 2 zusammen.

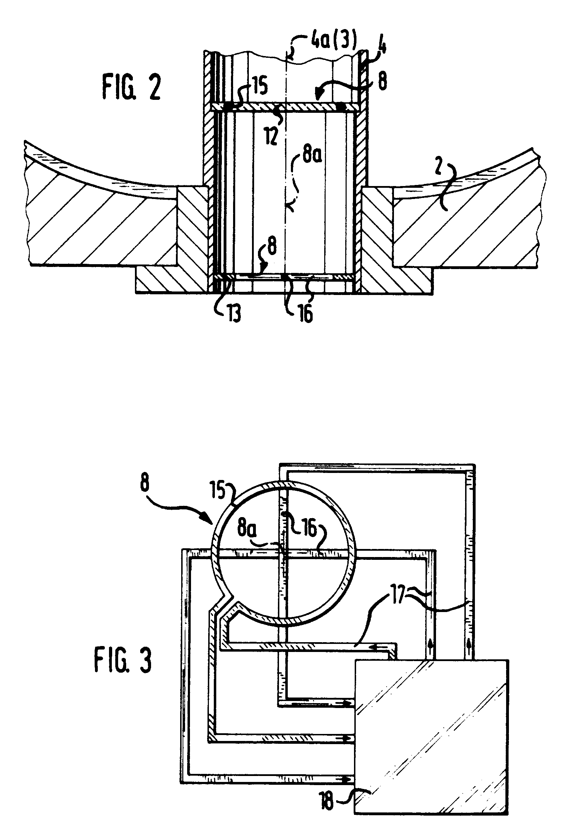

Der untere, dem Generator 2 zugekehrte Endbereich des Röntgentubus 4 ist mit einer eine Zielgerade 8a definierenden Zieleinrichtung 8 versehen, die in den Figuren 2 bis 5 näher gezeigt ist.

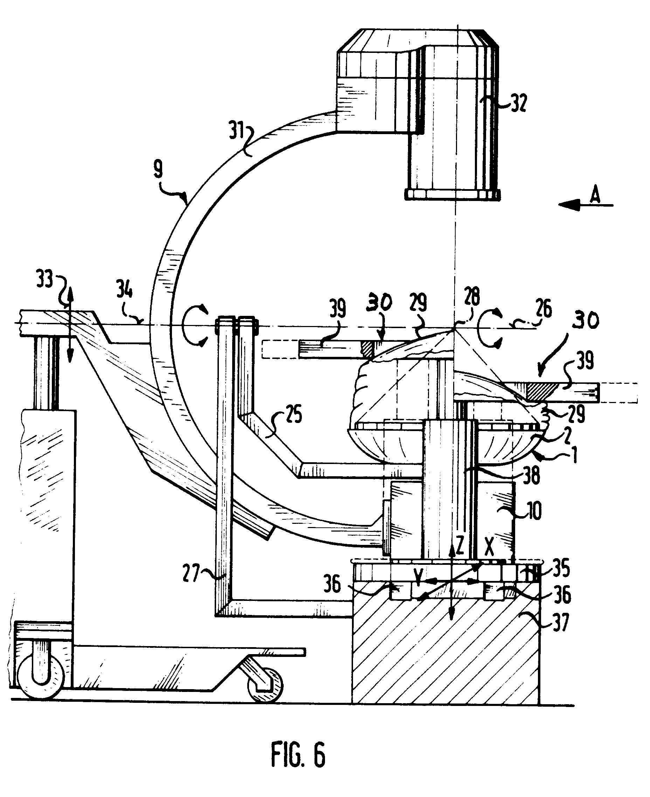

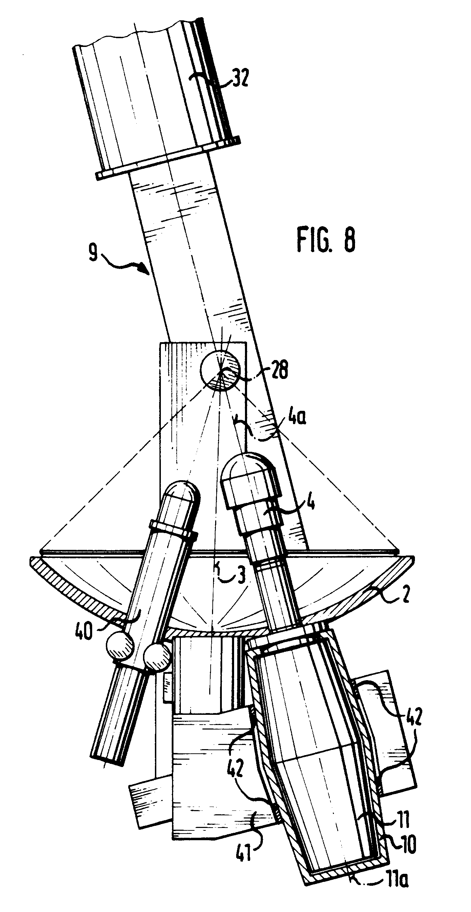

Unterhalb des Stoßwellengenerators 2 wird eine in den Figuren 6 und 8 besser zu erkennende, beistellbare Röntgeneinrichtung 9 der C-Arm-Bauart positioniert und fixiert, wenn eine Röntgenortung eines Konkrementes eines Patienten vorgenommen werden soll. Der besseren Übersicht wegen ist der Stoßwellengenerator 2 in Figur 1 nur teilweise dargestellt, d.h. es fehlt die flexible, obere Raumbegrenzung und das Flüssigkeitsmedium für die Übertragung der das Konkrement zerstörenden Schallwellen. Diese Merkmale sind dem Fachmann jedoch in Bau und Funktion bekannt. Die in Figur 1 ebenfalls teilweise dargestellte Röntgeneinrichtung 9 zeigt in dieser Figur nur eine Halterung 10 und einen darin gelagerten Röntgenstrahler 11.

Die Röntgeneinrichtung 9 der C-Arm-Bauart ist unterhalb des Stoßwellengenerators 2 mit diesem lösbar verbindbar, und zwar derart, daß der Röntgenstrahler 11 der Einrichtung 9 mit seiner zentralen Strahlungsachse 11a fluchtend mit der Zielgeraden 8a der Zieleinrichtung 8 ausgerichtet ist. Hierzu ist z.B. der Stoßwellengenerator 2 mit in Figur 8 schematisch angedeuteten Halte-, Justier- und Fixiermitteln 41,42 versehen, so daß der Röntgenstrahler 11 relativ zu dem Generator 2 justiert und dann fixiert werden kann. Nach der Fixierung bilden die Röntgeneinrichtung 9 und der Stoßwellengenerator 2 eine Einheit und sind gemeinsam konfokal um den Fokus des Stoßwellengenerators verschwenkbar.

Die Figuren 2 und 3 zeigen eine erste Ausführungsform der Zieleinrichtung 8. Sie besteht aus zwei im unteren Endbereich des Röntgentubus 4 vorgesehenen, voneinander mit Abstand und parallel zueinander angeordneten Scheiben 12 und 13 aus röntgennegativem Material, zum Beispiel aus Plexiglas, und aus Zielmarken 15,16 aus röntgenpositivem Material. Die Zielmarken können zum Beispiel aus Kanälen bestehen, die innerhalb der Scheiben ausgebildet sind und geometrische Figuren darstellen, derart, daß durch diese Figuren der Verlauf und die Lage einer gedachten Zielgeraden 8a definiert ist, die mit der Mittellängsachse 4a des Röntgentubus 4 zusammenfällt, die wiederum, wie bereits erwähnt, mit der zentralen Achse 3 des Stoßwellengenerators 2 zusammenfällt. Letzteres muß jedoch nicht unbedingt der Fall sein, wie Figur 8 zeigt, wenn nur gesichert ist, daß die Achse 4a des Röntgentubus 4 durch den Fokus des Generators 2 verläuft.

Die geometrischen Figuren der Zielmarken bestehen im dargestellten Beispiel aus einem Kreis 15 für die obere Scheibe 12 und aus einem Kreuz 16 für die untere Scheibe 13. Dies ist aus Figur 3 deutlich zu erkennen. Anstelle eines Kreuzes kann ebenfalls ein Kreis vorgesehen sein. Ferner können auch andere Figuren für die Zielmarken vorgesehen sein. Man erkennt ferner, daß die die Zielmarken 15 und 16 darstellenden Kanäle über Leitungen 17 zum Zu- und Abführen der röntgenpositiven Flüssigkeit mit einer Pumpeinrichtung 18 verbunden sind.

Die röntgenpositive Flüssigkeit wird in die die Zielmarken bildenden Kanäle gepumpt, wenn der Röntgenstrahler 11 der Röntgeneinrichtung 9 zum Röntgentubus 4 ausgerichtet und die Röntgenortung des Therapieobjektes vorgenommen werden soll. Es ist durch die Zieleinrichtung 8 die Zielgerade 8a vorgegeben, so daß der Röntgenstrahler 11 hierzu schnell und einfach eingestellt werden kann, d.h. das Zentrum des Strahlenbüschels des Röntgenstrahlers wird mit der Zielgeraden bzw. der Längsmittellinie 4a des Röntgentubus 4 in Überdeckung gebracht und damit auf den Fokus des Stoßwellengenerators ausgerichtet.

Die röntgenpositive Flüssigkeit kann aus einem bekannten Röntgenkontrastmittel oder auch aus einem Gemisch aus Alkohol und einem Röntgenkontrastmittel bestehen. Als weitere Alternative kann auch Quecksilber eingesetzt werden, wobei das Thermometerprinzip ausgenutzt wird.Das Ein- und Herausleiten des Quecksilbers wird hierbei vorzugsweise über Erwärmung oder Abkühlung mit Hilfe eines Peltierelementes durchgeführt.

Durch das Einführen und wieder Herausführen der röntgenpositiven Flüssigkeit in die bzw. aus den Kanälen werden diese sozusagen im Anwendungsfall in den Röntgentubus 4 eingeblendet bzw. danach wieder ausgeblendet. Anstelle einer Flüssigkeit für das röntgenpositive Material kann auch ein geeignetes Gas verwendet werden.

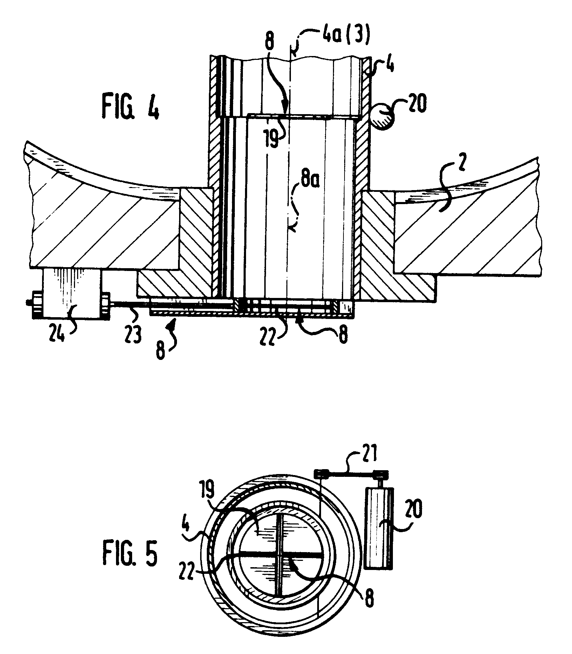

In den Figuren 4 und 5 ist eine mechanische Zieleinrichtung 8 dargestellt. Figur 4 zeigt, daß im unteren Endbereich des Röntgentubus 4 ein Metallring 19 als erste Zielmarke vorgesehen ist, dessen Mittelpunkt mit der Längsachse 4a des Tubus 4 zusammenfällt und der gemäß Figur 4 um eine horizontale Achse verdrehbar ausgebildet ist. Hierzu ist außerhalb des Tubus 4 ein Motor 20 vorgesehen, der über einen Zugmitteltrieb 21 die Verschwenkung des Metallringes 19 bewirkt. Am Unterende des Tubus 4 ist ein Metallteil 22 in Form eines Zielkreuzes als zweite Zielmarke vorgesehen, das mittels einer Antriebsstange 23 durch eine Hubeinrichtung 24, zum Beispiel ein Hubmagnet, horizontal verstellbar und einstellbar ist. Somit sind auch diese Zielmarken 19 und 22 im Bedarfsfall einblendbar und ausblendbar. Auch in diesem Fall sind die beiden Zielmarken 19 und 22 voneinander beabstandet und zueinander parallel angeordnet und bilden geometrische Zielfiguren der gewünschten Form.

Figur 6 zeigt, daß das Therapiegerät 1 mittels eines Armes 25 um eine horizontale Achse 26 schwenkbar an einem Gestell 27 gelagert ist. Die horizontale Achse 26 verläuft dabei durch den Fokus 28 des Stoßwellengenerators 2. Der Ankoppelballon des Generators 2 ist mit 29 bezeichnet. Im linken Teil des Stoßwellengenerators 2 ist der Ballon 29 ausgefahren gezeigt. Ebenfalls ist der Patientenliegetisch 30 in einer abgesenkten und in einer erhöhten Lage gezeigt.

Die beistellbare Rönteneinrichtung 9 umfaßt einen C-förmigen Trägerarm 31, dessen eines Ende den bereits erwähnten Röntgenstrahler 11 und dessen anderes Ende einen Bildverstärker 32 aufweist, der mit dem Röntenstrahler fluchtet. Der C-förmige Trägerarm ist gemäß dem Doppelpfeil 33 in der Höhe einstellbar. Im gezeigten Fall ist die Röntgeneinrichtung 9 so eingestellt, daß deren horizontale Schwenkachse 34 mit der horizontalen Schwenkachse 26 des Stoßwellengenerators 2 zusammenfällt. Somit verläuft auch die Schwenkachse 34 durch den Fokus 28 des Generators 2, wodurch auch die Röntgeneinrichtung 9 konfokal um den Fokus 28 verschwenkbar ist.

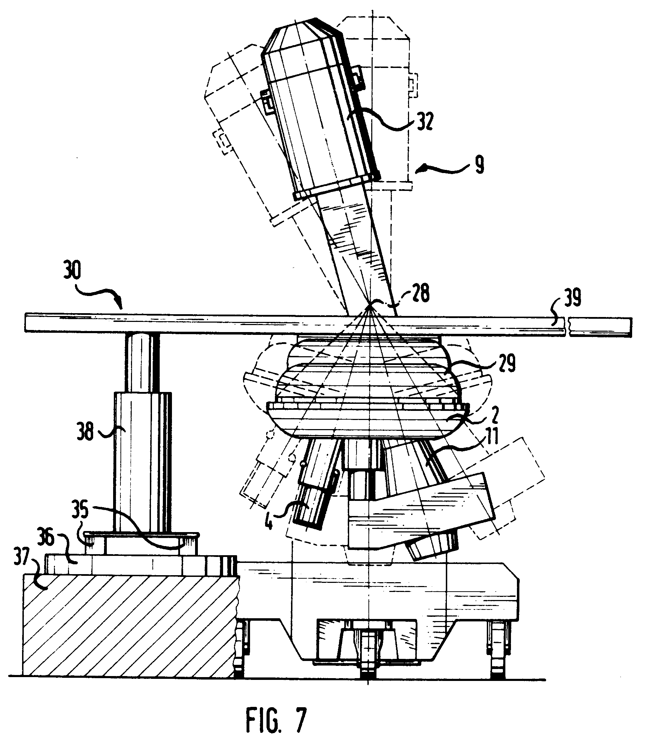

Aus Figur 7 ist der Patientenliegetisch 30 am besten zu erkennen. Dieser Tisch ist in üblicher Weise in den drei in Figur 6 angedeuteten Raumkoordinaten X, Y und Z verstellbar. Die Einstelleinrichtung hierfür besteht aus zwei unteren, flachbauenden, in den horizontalen X-Y-Koordinaten einstellbaren Stelleinheiten 35 und 36, die auf einem Sockel 37 ruhen, und aus einer auf der oberen Einheit 35 angeordneten Hubeinheit 38, die oben mit der Tischplatte 39 verbunden ist. Die Konstruktion dieser Einstelleinrichtung, d.h. die Anordnung der Einheiten 35,36 in der Planebene unterhalb der Hubeinheit 38, ermöglicht, das Vorsehen einer relativ dünn gestalteten Tischplatte, was einen vergrößerten Freiraum unterhalb der Tischplatte ergibt. Daraus wiederum resultiert ein größerer Schwenkwinkelbereich für das Therapiegerät unterhalb der Tischplatte 39.

Figur 8 zeigt, daß der Röntgentubus 4 auch außerhalb der zentralen Achse 3 des Stoßwellengenerators 2 angeordnet sein kann. Seine Mittellängsachse 4a befindet sich im Winkel zur genannten Achse 3. Zusätzlich kann ein weiteres Ortungsgerät 40 vorgesehen sein, das nach dem kontinuierlichen Ultraschall-Ortungsprinzip arbeitet. Dieses Gerät ist mit seiner Längsachse ebenfalls im Winkel zu der zentralen Achse 3 angeordnet. Durch diese Ausbildung des Therapiegerätes mit zusätzlichem Ultraschallortungssystem ist auch eine craniale/caudale Röntgenortung möglich, z.B. im Winkel von 15° zur zentralen Achse 3.

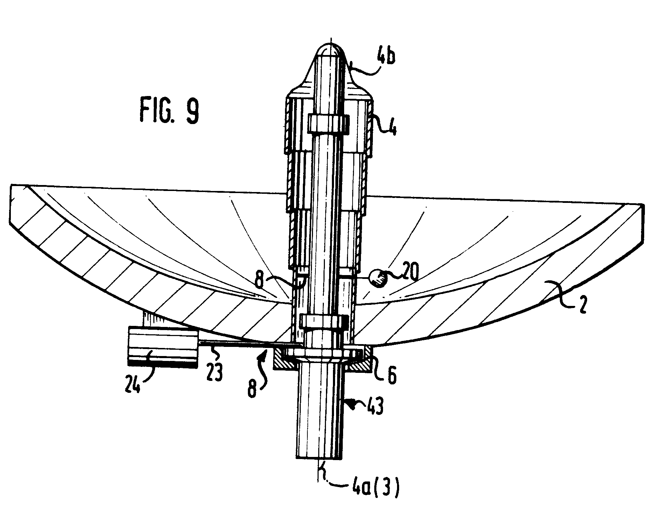

Figur 9 zeigt eine abgeänderte Ausführungsform des Therapiegerätes zur Ortung des Therapieobjektes. Anstelle der Röntgenortung kann eine Ultraschallortung angewendet werden. Hierzu werden die Röntgeneinrichtung 9, das Plattenteil 7 und die Zieleinrichtung 8 (Fig. 1) entfernt und ein Ultraschall-Ortungsscanner 43 in den Röntgentubus 4 eingesetzt und an dem Fassungsteil 6 montiert. Der Scanner 43 ist an dem Fassungsteil 6 drehbar und höhenverstellbar, d.h. um die und in Richtung der Längsachse 4a des Röntgentubus 4, gelagert. Diese Bewegungen können motorisch ausgeführt werden. Der Röntgentubus 4 weist ein flexibles Abschlußteil 4b aus z.B. Latex auf. Der Scanner 43 ist über eine Gelmasse ultraschallmäßig mit dem Patienten gekoppelt ist. Mittels des Ultraschallgenerators 43 wird nach erfolgter Ultraschallortung des Therapieobjektes die Behandlung des Objektes, z.B. ein Gewebe, vorgenommen.

## Patentansprüche

1. Extrakorporales Therapiegerät zum Behandeln von Konkrementen und Gewebe mittels Schallwellen im Körper eines Patienten, mit einer Generatoreinrichtung zum Erzeugen der Schallwellen, mit einer Zieleinrichtung und unter Verwendung einer beistellbaren Röntgeneinrichtung zum Orten eines Therapieobjektes, dadurch gekennzeichnet, daß die Zieleinrichtung (8) dem Durchgang eines an der Generatoreinrichtung (2) befestigten und mit seiner zentralen Längsachse (4a) auf deren Fokus (28) ausgerichteten Röntgentubus (4) für die beistellbare, an der Generatoreinrichtung lösbar fixierbare Röntgeneinrichtung (9) zugeordnet ist.

2. Therapiegerät nach Anspruch 1, dadurch gekennzeichnet, daß die Zieleinrichtung (8) aus zwei im Innern des Röntgentubus (4) voneinander mit Abstand und parallel zueinander angeordneten Scheiben (12,13) aus röntgennegativem Material und aus an den Scheiben vorgesehenen Zielmarken (15,16) aus röntgenpositivem Material besteht.

3. Therapiegerät nach Anspruch 2, dadurch gekennzeichnet, daß die Zielmarken (15,16) aus in den Scheiben (12,13) ausgebildeten, geometrische Figuren darstellenden Kanälen und aus einer durch die Kanäle pumpbaren, röntgenpositiven Flüssigkeit bestehen.

4. Therapiegerät nach Anspruch 3, dadurch gekennzeichnet, daß die röntgenpositive Flüssigkeit ein Röntgenkontrastmittel, Quecksilber oder ein Gemisch aus Alkohol und einem Röntgenkontrastmittel ist.

5. Therapiegerät nach Anspruch 1, dadurch gekennzeichnet, daß die Zieleinrichtung (8) aus zwei voneinander beabstandeten, zueinander parallelen und geometrisch geformten Zielfiguren aus festem röntgenpositivem Material (19,22) besteht.

6. Therapiegerät nach Anspruch 5, dadurch gekennzeichnet, daß die Zielfiguren aus Metallbauteilen (19,22) bestehen und aus dem den Röntgentubus (4) passierenden Strahlengang der Röntgeneinrichtung (9) heraus- und darin hineinschwenkbar sind.

7. Therapiegerät nach Anspruch 1, mit einem Stoßwellengenerator der Kalottenbauart, dadurch gekennzeichnet, daß der Röntgentubus (4) im Zentrum des Kalottenbauteils oder dazu winkelversetzt fest angeordnet ist, derart, daß die Zielgerade der Zieleinrichtung (8) durch den Fokus des Stoßwellengenerators (2) verläuft.

8. Therapiegerät nach Anspruch 1, dadurch gekennzeichnet, daß in den Röntgentubus (4) ein Ultraschallscanner (42) zur Ultraschallortung einsetzbar ist, der motorisch drehbar und höhenverstellbar ausgebildet ist.

9. Therapiegerät nach Anspruch 1, mit einem Patientenliegetisch mit einer Einrichtung zu dessen Einstellung in drei Raumkoordinaten, dadurch gekennzeichnet, daß die den Tisch (39) tragende Einstelleinrichtung aus zwei unteren, flachbauenden, in den horizontalen X-Y-Koordinaten einstellbaren Stelleinheiten (35,36) aus einer darauf angeordneten, die Tischplatte tragenden Hubeinheit (38) besteht.

10. Therapiegerät nach einem der Ansprüche 2 bis 9, dadurch gekennzeichnet, daß die Mittelpunkte der Scheiben (12,13) auf der Mittelachse des Röntgentubus liegen.
